# EUROPÄISCHE PATENTANMELDUNG

(11) **EP 3 973 911 A1**
(43) Veröffentlichungstag der Anmeldung: **30.03.2022**
(21) Anmeldenummer: 20198424.2
(22) Anmeldetag: 25.09.2020
(51) Int. Cl.: A61B 90/00, A61F 11/00, A61F 5/01

(54) **OHRFORMUNGSSCHIENE UND VERFAHREN ZUR HERSTELLUNG DAVON**

(71) Anmelder: Brom, Jörn, 69115 Heidelberg (DE); Spahn, Sonja, 63179 Oberhausen (DE)
(72) Erfinder: Brom, Jörn, 69115 Heidelberg (DE); Spahn, Sonja, 63179 Oberhausen (DE)
(74) Vertreter: Lederer & Keller Patentanwälte Partnerschaft mbB

(57) **Zusammenfassung**

Die Erfindung bezieht sich auf eine Vorrichtung zur Formanpassung einer fehlgebildeten Ohrmuschel. Solche Vorrichtungen werden auch als Ohrformungsschienen bezeichnet. Insbesondere bezieht sich die Erfindung auf eine Ohrformungsschiene zur Formanpassung einer fehlgebildeten Ohrmuschel bei menschlichen Neugeborenen oder Säuglingen. Außerdem bezieht sich die Erfindung auf einen Herstellungsprozess für eine solche Schiene.

## Beschreibung

### TECHNISCHES GEBIET

Die Erfindung bezieht sich auf eine individuell angepasste Vorrichtung zur Formanpassung einer fehlgebildeten Ohrmuschel. Die Formanpassung einer fehlgebildeten Ohrmuschel wird auch als "Earmolding" bezeichnet. Insbesondere bezieht sich die Erfindung auf eine Ohrformungsschiene zur Formanpassung einer fehlgebildeten Ohrmuschel bei menschlichen Neugeborenen oder Säuglingen. Außerdem bezieht sich die Erfindung auf einen Herstellungsprozess für eine solche Schiene.

### HINTERGRUND

Angeborene Fehlbildungen (Dysplasien) des Ohres treten bei etwa 0,76 bis 2,35 von 10.000 Geburten in unterschiedlichen Ausprägungen auf. Legt man die aktuelle Geburtenrate zugrunde, kommen in Deutschland etwa 100 bis 150 neue Fälle pro Jahr hinzu. Die Verformungen reichen von einfach abstehenden Ohren bis hin zu vollkommenem Fehlen der Ohrmuschel (*Auricula auris*).

Die Ohrmuschel ist ein Teil des Außenohres und ist mit dem *Periost* des menschlichen Schädels (*Perikranium*) verwachsen. Die Form der Ohrmuschel wird durch den Ohrknorpel (*Cartilago auriculae*) geprägt. Der Ohrknorpel besteht aus einem Stück, ist elastisch und stark gefaltet. Die Falten bilden das typische Ohrrelief mit zahlreichen Erhebungen und Vertiefungen (Faltenstruktur der Ohrmuschel), die jeweils eigene Bezeichnungen tragen. Die Ohrmuschel weist eine Vorder- und eine Rückseite auf. Der prominente äußere Rand der Ohrmuschel wird als *Helix* bezeichnet. Die Helix verläuft an der äußeren Seite der Ohrmuschel relative gerade von oben nach unten und in ihrem oberen Teil bogenförmig auf den Schädel zu. Parallel zur *Helix* auf der Ohrmuschelvorderseite, getrennt durch eine gekrümmte rinnenförmige Einsenkung, die sogenannte *Scapha*, verläuft als prominenter Wulst die sichelförmige *Anthelix.* Die *Anthelix* verzweigt sich im oberen Drittel der Ohrmuschel in einen oberen (*Crus superius anthelices*) und einen unteren Schenkel (*Crus inferius anthelicis*)*.* Zwischen diesen beiden Schenkeln liegt eine dreieckige Einziehung, die *Fossa triangularis.* Die *Anthelix* rahmt die eigentliche Ohrmuschel (*Concha auricularis*) ein, eine ausgedehnte Vertiefung. Diese ausgedehnte Vertiefung, auch als Ohrhöhlung bezeichnet, führt über den Gehörgangstrichter (*Cavum conchae*) zum äußeren Gehörgang (*Meatus acusticus externus*)*.* Der den Eingang zum Gehörgang überlappende Höcker wird als Ohrdeckel (*Tragus*) bezeichnet. Vorn-seitlich gegenüber dem *Tragus* liegt der *Antitragus.* Das Ohrläppchen (*Lobulus auriculae*) ist der weiche Teil der unteren Ohrmuschel, der nicht vom Ohrknorpel gestützt wird. Die wichtigste Vertiefung der Ohrmuschelrückseite (*Dorsum auriculae*) ist die von oben nach unten und fast über die gesamte Länge des Ohres verlaufende retroauriculäre *Sulcus* (*Fossa anthelicis*)*.* Die Vertiefung entspricht der Rückseite der *Anthelix* und kann recht ausgeprägt sein.

Bei der Einteilung der Ohrmuschelfehlbildungen werden drei Grade unterschieden:
- Bei der Dysplasie 1 sind die meisten anatomischen Strukturen der Ohrmuschel, wie oben beschrieben, vorhanden. Hierzu zählen u.a. abstehende Ohren (*Apostasis otum*), Tassenohr I und Tassenohr II.
- Bei der Dysplasie 2 sind nur einige Strukturen der anatomisch korrekten geformten Ohrmuschel vorhanden. Hierzu zählen u.a. Tassenohr III und das Miniohr.
- Bei der Dysplasie 3 sind die oben genannten Ohrstrukturen praktisch nicht vorhanden, es existieren lediglich Rudimente. Hierzu zählen u.a. *Mikrotie III*, *Anotie* und *Dystopie.*

Beim Menschen beginnt die Ohrentwicklung in einem sehr frühen embryonalen Stadium, d.h. in der 3. bis 6. embryonalen Entwicklungswoche. Die Ausbildung der oben beschriebenen Form und Faltungen der Ohrmuschel, d.h. die feine Ohrstruktur, beginnt jedoch erst am Ende des 6. Fetalmonats. Der Ohrknorpel eines Neugeborenen bzw. Säuglings ist häufig noch sehr weich, flexibel und formbar, da noch bis zur 6. Lebenswoche des Kindes das mütterliche Östrogen im Blutkreislauf des Säuglings zirkuliert. Es hat sich herausgestellt, dass aufgrund der Weichheit des Ohrknorpels während dieser sensiblen Entwicklungsphase des Kindes Ohrdeformationen, insbesondere mit einem Dysplasie-Grad von 1, einfach und dauerhaft durch ein formgebendes Verfahren, das heißt ohne chirurgischen Eingriff, korrigiert werden können. Dabei gibt das Alter des Säuglings bei Beginn der Behandlung die ungefähre Therapiedauer vor. Eine frühzeitige Behandlung nach der Geburt erzielt den größten Erfolg und ist daher wünschenswert. Amerikanische Studien belegen eine Erfolgsquote von 90 %, wenn die Behandlung frühzeitig begonnen wird und Patientenauswahl korrekt erfolgt (siehe z.B. Anstadt et. al. Pediatrics, Vol. 137, Nr. 3, März 2016, Case Report).

Im Stand der Technik sind bereits Vorrichtungen zur Formanpassung einer fehlgebildeten Ohrmuschel, insbesondere eines Neugeborenen oder Säuglings, bekannt.

So wird in US 2014/0378879 A1 eine Vorrichtung zur Formanpassung einer fehlgebildeten Ohrmuschel beschrieben, die aus einem Basisstück, einer Schiene und mindestens einer Klammer besteht. Das Basisstück der Vorrichtung enthält eine Öffnung, die so bemessen ist, dass die zu behandelnde Ohrmuschel durch die Öffnung passt. Das Basisstück wird mittels Klebeband periaurikulär befestigt. Zur Behandlung der Ohrmuschelfehlbildung wird die Schiene in der Ohrmuschelvorderseite so positioniert, so dass sie sich zwischen *Anthelix* und *Crus superius anthelices* befindet. Des Weiteren werden Klammern verwendet, die gewährleisten sollen, dass während des Wachstums der Ohrmuschel diese in die anatomisch korrekte Form der *Scapha* geformt wird bzw. diese beibehält.

Des Weiteren sind im Stand der Technik Schienen bekannt, die die anatomisch korrekte Form der sichelförmigen *Anthehelix* aufweisen und mittels Klebebänder im Bereich der Scapha platziert werden, um so Dysplasien bei Säuglingen zu behandeln (siehe zum Beispiel Smith et. al, "Nonsurgical correction of congenital earabnormalities in the newborn. Case series", Paediatr. Child Health, Vol. 10, Nr.6, Juli/August 2005, Seiten 327-331; oder www.earbudies.co.uk).

Diese aus dem Stand der Technik bekannten Vorrichtungen weisen jedoch diverse Nachteile auf. So können die verwendeten Schienen falsch angebracht werden, so dass die Fehlbildungen nicht anatomisch korrekt behoben werden oder sogar fehlgeformt werden. Die Verwendung von Klammern und/oder Klebebändern erschweren das Anlegen und Ablegen der Vorrichtung an der zu behandelnden Ohrmuschel. Außerdem können beim Ablegen der Vorrichtung Kleberreste am Kopf des Säuglings zurückbleiben, was ebenfalls nicht erwünscht ist.

Daher war es Aufgabe der vorliegenden Erfindung eine Vorrichtung zur Verfügung zu stellen, die einfach angelegt und abgelegt werden kann und keine wesentlichen Einschränkungen in der Alltagsanwendung mit sich bringt. Keine Klammern und/oder Kleberstreifen benötigt, deren Verwendung, wie oben beschrieben, ebenfalls von Nachteil sind. Außerdem sollte die Vorrichtung so konstruiert sein, dass sichergestellt werden kann, dass auch nicht-medizinisch fachkundige Personen, z.B. die Eltern des zu behandeln Säuglings, diese in korrekte Art und Weise an die zu behandelnde Ohrmuschel anlegen können. Des Weiteren sollte die Vorrichtung einfach und kostengünstig herstellbar sein.

### ZUSAMMENFASSENDE BESCHREIBUNG DER ERFINDUNG

Die Erfinder der vorliegenden Anmeldung haben herausgefunden, dass die oben beschriebenen Aufgaben durch folgende Vorrichtung gelöst werden können:
Vorrichtung zur Formanpassung einer fehlgebildeten Ohrmuschel umfassend
   a. ein erstes Formteil (1) mit einer Vorderseite und einer Rückseite,
   b. ein zweites Formteil (2) mit einer Vorderseite und einer Rückseite, und
   c. ein Verschlusssystem umfassend mindestens zwei Verschlusselemente,
dadurch gekennzeichnet, dass
   - die Vorderseite des ersten Formteils (1) die Negativform einer gewünschten anatomischen Form eines Teils der Ohrmuschelvorderseite aufweist,
   - Die Vorderseite des zweiten Formteils (2) die Negativform einer gewünschten anatomischen Form eines Teils der Ohrmuschelrückseite aufweist, und
   - das Verschlusssystem das erste Formteil (1) und das zweite Formteil (2) reversibel miteinander verbinden kann, so dass die fehlgebildete Ohrmuschel zwischen beiden Formteilen (1; 2) positioniert werden kann.

Des Weiteren stellt die vorliegende Erfindung ein Herstellungsverfahren für die erfindungsgemäße Vorrichtung zur Verfügung, das folgende Schritte umfasst:
a) Gegebenenfalls Erstellen oder Bereitstellen eines Modells der fehlgebildeten Ohrmuschel;
b) Bearbeiten des in Schritt a) erhaltenen Modells, so dass ein Modell einer Ohrmuschel mit gewünschter anatomischer Form erhalten wird;
c) Herstellen eines ersten Formteils, das die Negativform eines Teils der Ohrmuschelvorderseite des in Schritt b) erhaltenen Modells aufweist und wenigstens ein Verschlusselement umfasst; und
d) Herstellen eines zweiten Formteils, das die Negativform eines Teils Ohrmuschelrückseite des in Schritt b) erhaltenen Modells aufweist und wenigstens ein Verschlusselement umfasst;
wobei das erste Formteil und das zweite Formteil reversibel miteinander verbunden werden können, so dass die fehlgebildete Ohrmuschel zwischen beiden Formteilen positioniert werden kann.

Die erfindungsgemäße Vorrichtung soll insbesondere zur Behandlung von Dysplasien bei Neugeborenen oder Säuglingen verwendet werden. Die erfindungsgemäße Vorrichtung ist daher so dimensioniert, dass damit ein Neugeborenes oder ein Säugling behandelt werden kann.

Die erfindungsgemäße Vorrichtung kann einfach angelegt und abgelegt werden. Durch das vorhandene Verschlusssystem ist die Verwendung von zusätzlichen Klammern und/oder Klebebändern zur Fixierung der Vorrichtung an der Ohrmuschel des zu behandelnden Säuglings nicht notwendig. Das erfindungsgemäße Herstellungsverfahren gewährleistet, dass die Vorrichtung eine optimale Passform aufweist und so keine Druckstellen beim Tragen der Vorrichtung entstehen. Insbesondere wird durch die Anfertigung einer individuellen Ohrformungsschiene gemäß Erfindung gewährleistet, dass diese auch z.B. von den Eltern des Säuglings in korrekter Art und Weise an der zu behandelnden Ohrmuschel angebracht werden kann. Die erfindungsmäße Vorrichtung kann kostengünstig hergestellt werden, insbesondere im Vergleich zu den Kosten, die durch einen operativen Eingriff zur Behandlung der Dysplasie ansonsten entstehen würden. Darüber hinaus sind mit einem chirurgischen Eingriff immer Risiken verbunden, die durch die Erfindung vermieden werden können.

### BESCHREIBUNG DER FIGUREN

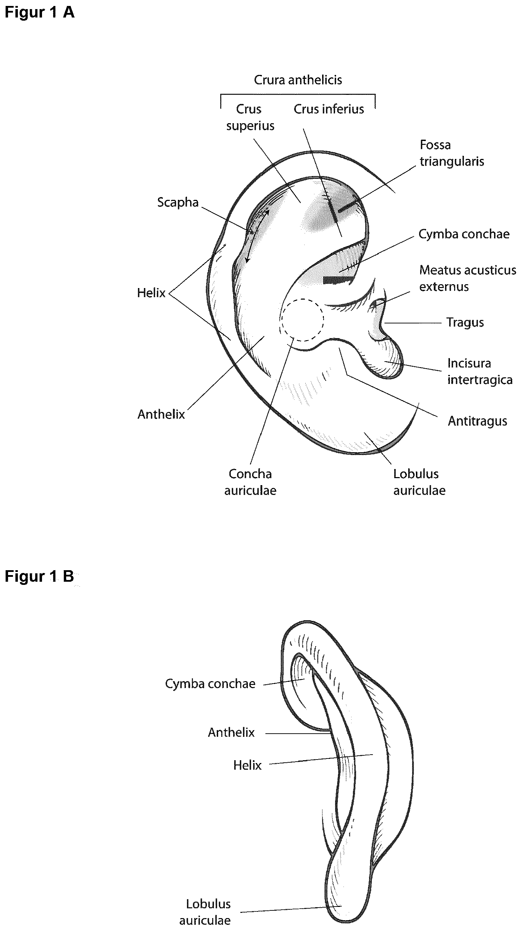

Die Figuren 1 A (Frontalansicht) und 1 B (Ansicht von dorsolateral) zeigen die Bezeichnungen von Teilen der menschlichen Ohrmuschel.

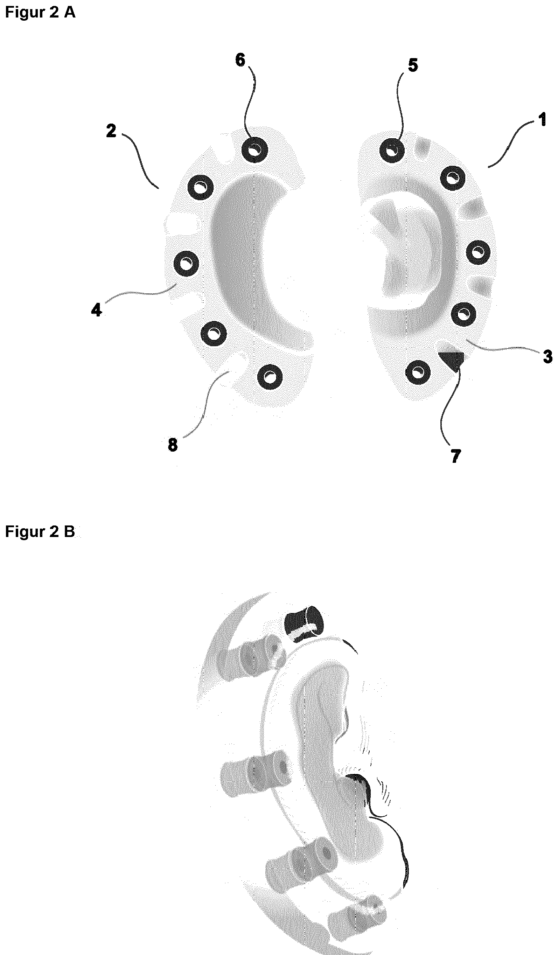

Figur 2 A zeigt das erste Formteil (rechts) und zweite Formteil (links) der erfindungsgemäßen Vorrichtung in getrennter Konfiguration. Figur 2 B zeigt die erfindungsgemäße Vorrichtung in geschlossener Konfiguration, wobei die Verschlusselemente des ersten Formteils und die Verschlusselement des zweiten Formteils die beiden Formteile miteinander verbinden.
Figur 3 zeigt sowohl die verschiedenen Modelle, die während des erfindungsgemäßen Herstellungsprozess angefertigt werden, als auch die fertige erfindungsgemäße Vorrichtung. Von links nach rechts ist in der Figur 3 A
   - ein Wachsmodell der fehlgebildeten Ohrmuschel,
   - ein Wachsmodell, das so moduliert wurde, dass es die gewünschte anatomische Form der Ohrmuschel aufweist und
   - das entsprechende Gipsmodell des modulierten Wachsmodells
   zu sehen. In den Abbildungen B der Figur 3 sieht man links ein Gipsmodell der anatomisch korrekten Ohrmuschel und rechts die auf Basis des Gipsmodells hergestellte erfindungsgemäße Vorrichtung.

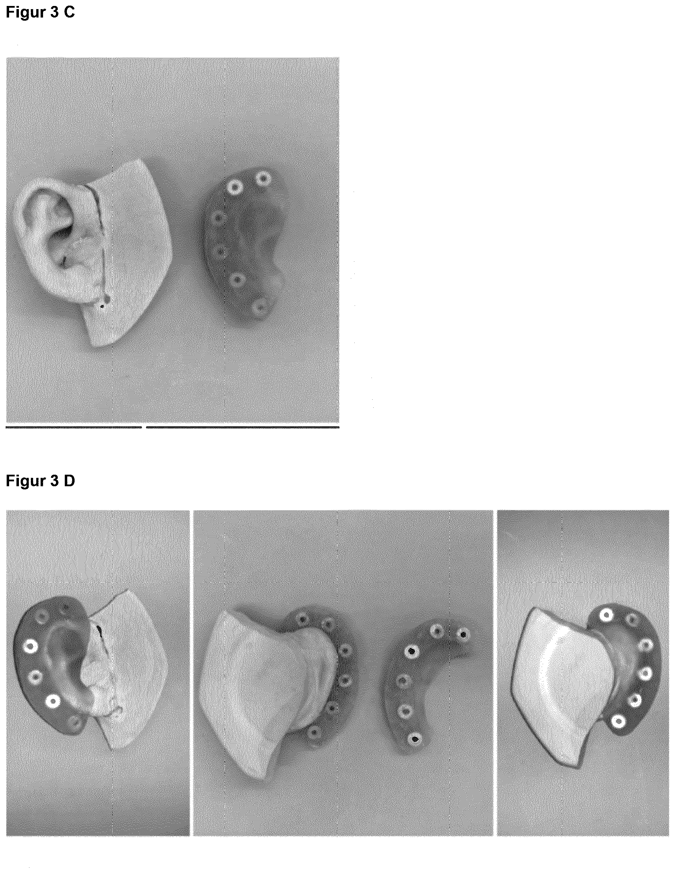

Figur 3 C und Figur 3 D zeigen verschiedene Ansichten der erfindungsgemäßen Vorrichtung.

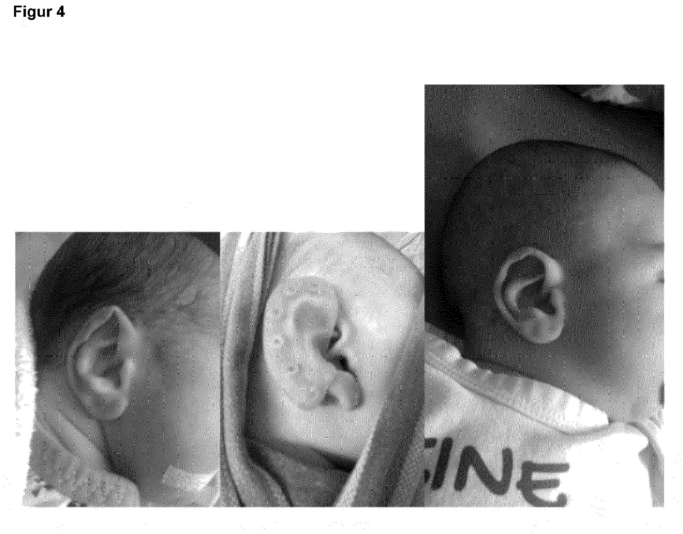

Figur 4 zeigt den Behandlungserfolg durch die Anwendung der erfindungsgemäßen Vorrichtung an einem Säugling mit Ohrmuschelfehlbildung. Links ist eine fehlgebildete Ohrmuschel abgebildet. In der Mitte ist die Ohrmuschel mit angebrachter Vorrichtung zu sehen. Rechts ist die Ohrmuschel nach der Behandlung durch die erfindungsgemäße Vorrichtung abgebildet.

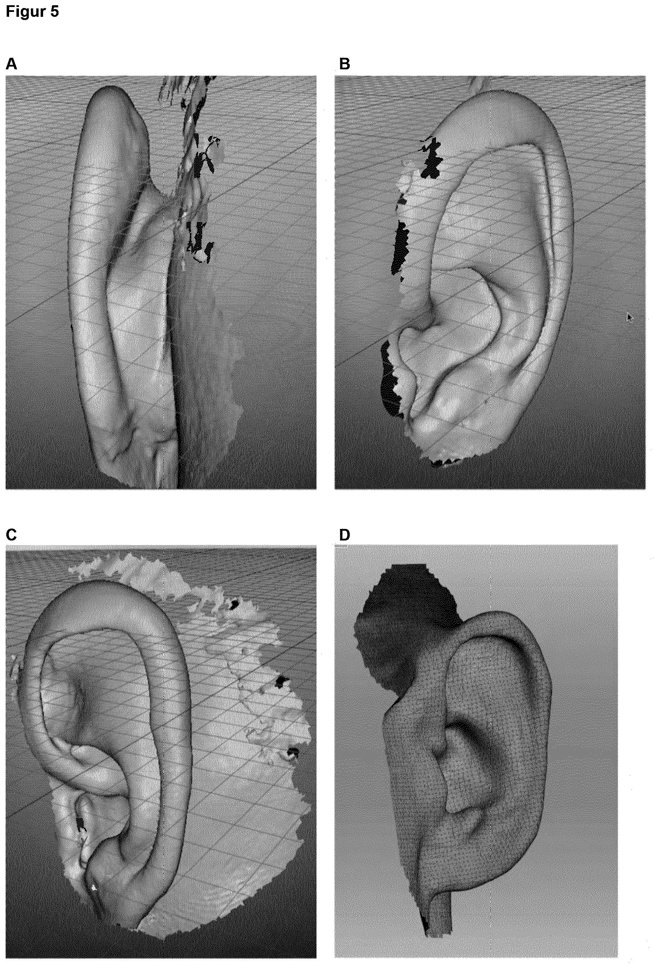

Die Figuren 5 A, B und C zeigen in unterschiedlichen Ansichten einen digitalen 3D-Scan eines linken, menschlichen Ohres, aufgenommen mit dem Artec 3D Space Spider (Fa. KLIB, 57635 Hasselbach), einem Farbscanner, der die Blaulicht-Technologie nutzt.
Figur 5 D zeigt die digitale Ansicht des linken Ohres nach 3D-Scan im Rasterfeld in Verwendung der Scansoftware Artec-Studio Version 14.

### DETAILLERTE BESCHREIBUNG DER ERFINDUNG

Bevor die Erfindung näher beschrieben wird, sollen im Folgenden einige Begriffe, wie sie in der Anmeldung verwendet werden, erläutert werden.

Als "Faltenstruktur" wird die durch die zahlreichen Erhebungen und Vertiefungen, die in der Ohrmuschel vorhanden sind, entstehende Struktur (Ohrrelief) bezeichnet.

Der Begriff "gewünschte anatomische Form und Faltenstruktur" bezieht sich in der vorliegenden Anmeldung vorzugsweise auf die aus medizinischer Sicht anatomisch korrekte Form und Faltenstruktur der Ohrmuschel, d.h. vorzugsweise auf eine Ohrmuschel ohne Fehlbildung. Dies gilt sowohl für die Vorder- als auch für die Rückseite der Ohrmuschel.

Ein biokompatibles Material ist ein Material, das nach ISO 10993 1-20 zertifiziert ist. Insbesondere versteht man in der vorliegenden Anmeldung unter dem Begriff "biokompatibles Material" ein Material, das keinen negativen Einfluss auf den zu behandelnden Säugling hat.

Als Säugling bezeichnet man ein Kind im 1. Lebensjahr, wobei ein Säugling in den ersten vier Lebenswochen auch als Neugeborenes bezeichnet wird.

Die erfindungsgemäße Vorrichtung zur Formanpassung einer fehlgebildeten Ohrmuschel umfasst ein erstes Formteil (1), ein zweites Formteil (2) und ein Verschlusssystem, das das erste und zweite Formteil (1; 2) reversibel miteinander verbindet, und zwar so, dass die fehlgebildete Ohrmuschel zwischen beiden Formteilen (1; 2) positioniert werden kann.

Das erste Formteil (1) der erfindungsgemäßen Vorrichtung weist die Negativform oder die Form eines Abdrucks der gewünschten anatomischen Form und Faltenstruktur der Ohrmuschelvorderseite oder eines Teils davon auf. Der Teil umfasst in der Regel *Helix, Scapha, Anthelix, Crus superius anthelicis, Crus inferius anthelices, Triangular fossa, Concha auriculae* und *Antitragus.* Mit anderen Worten, das erste Formteil (1) gemäß Erfindung bildet die Form und Faltenstruktur eines wesentlichen Teils der Ohrmuschelvorderseite ab. Dabei werden insbesondere folgende Komponenten des Ohrreliefs abgebildet: *Helix*, *Scapha*, *Anthelix*, *Crus superius anthelicis*, *Crus inferius anthelices*, *Triangular fossa*, *Concha auriculae* und *Antitragus.*

In einer besonderen Ausführungsform der Erfindung umfasst die Faltenstruktur des Formteils (1) nicht das *Cavum conchae* der Ohrmuschelvorderseite. In einer anderen Ausführungsform umfasst die Faltenstruktur des Formteils (1) nicht den *Tragus* der Ohrmuschelvorderseite. In einer anderen Ausführungsform umfasst die Faltenstruktur des Formteils (1) nicht den *Lobulus auriculae* der Ohrmuschelvorderseite. In einer weiteren Ausführungsform umfasst die Faltenstruktur des Formteils (1) nicht das *Cavum conchae* der Ohrmuschelvorderseite und nicht den *Tragus* der Ohrmuschelvorderseite. Damit wird sichergestellt, dass die Hörfunktion des zu behandelnden Ohres durch die verwendete Vorrichtung nicht beeinträchtigt wird. In einer weiteren Ausführungsform umfasst die Faltenstruktur des Formteils (1) nicht den *Lobulus auriculae* der Ohrmuschelvorderseite und nicht den *Tragus* der Ohrmuschelvorderseite. In einer weiteren Ausführungsform umfasst die Faltenstruktur des Formteils (1) nicht den *Lobulus auriculae* der Ohrmuschelvorderseite, nicht das *Cavum conchae* der Ohrmuschelvorderseite und nicht den *Tragus* der Ohrmuschelvorderseite.

Das zweite Formteil (2) gemäß Erfindung weist die gewünschte Form und Faltenstruktur, insbesondere die *Fossa anthelicis,* des Ohrmuschelrückens auf.

Gemäß Erfindung ist es bevorzugt, dass sowohl das erste Formteil (1) als auch das zweite Formteil (2) der erfindungsgemäßen Vorrichtung jeweils aus einem Stück bestehen.

Darüber hinaus ist es bevorzugt, dass die Formteile (1) und (2) so ausgestaltet sind, dass das erste Formteil (1) außerhalb des Bereichs, der der *Helix* entspricht, einen Rand aufweist, und dass das zweite Formteil (2) einen eben solchen Rand aufweist. Diese Ränder weisen nicht die oben beschriebenen Faltenstrukturen der Ohrmuschel Vorder- und Rückseite auf. Bei vorgesehener Anwendung der Vorrichtung wird der Rand des ersten Formteils mit dem Rand des zweiten Formteils in Kontakt gebracht, wobei die beiden Formteile das zu behandelnde Ohr umschließen. In dieser "geschlossenen Konfiguration" ist die Vorderseite des Rands des ersten Formteils (1) vorzugsweise in engem Kontakt mit der Vorderseite des Rands des zweiten Formteils (2). Das wird dadurch erreicht, dass der Rand des ersten Formteils über seine gesamte Länge komplementär zum Rand des zweiten Formteils (2) geformt ist, so dass die beiden Ränder in der geschlossenen Konfiguration komplementär ineinander greifen oder sich über die gesamte Länge der Ränder berühren. Die Ränder sind vorzugsweise im Wesentlichen flächig ausgebildet. Der Rand des ersten Formteils kann eine oder mehrere Erhebung(en) und/oder Vertiefung(en) aufweisen, zum Beispiel eine, zwei, drei, vier oder fünf Erhebung(en) und/oder Vertiefung(en). Der Rand des zweiten Formteils kann eine oder mehrere Erhebung(en) und/oder Vertiefung(en) aufweisen, zum Beispiel eine, zwei, drei, vier oder fünf Erhebung(en) und/oder Vertiefung(en). Bei Verwendung eines Pilzverschlusssystems kann beispielsweise eine einzige Erhöhung bzw. Vertiefung je Formteil ausreichen. Bei Verwendung eines Magnetverschlusssystems wird jedes Formteil in der Regel mehrere Erhöhungen oder Vertiefungen aufweisen. Wenn der Rand des ersten Formteils an einer Stelle eine Erhebung aufweist, hat der Rand des zweiten Formteils an der entsprechenden Stelle eine komplementär ausgebildete Vertiefung, so dass die Erhebung und die Vertiefung bei funktionsgemäßer Verwendung der Vorrichtung komplementär ineinander greifen. Wenn der Rand des ersten Formteils an einer Stelle eine Vertiefung aufweist, hat der Rand des zweiten Formteils an der entsprechenden Stelle eine komplementär ausgebildete Erhebung, so dass die Erhebung und die Vertiefung bei funktionsgemäßer Verwendung der Vorrichtung komplementär ineinander greifen. Dadurch wird vermieden, dass sich die beiden Formteile bei geschlossener Konfiguration gegeneinander verschieben.

Es ist weiterhin bevorzugt, dass beide Formteile (1, 2) im Wesentlichen aus einem biokompatiblen Material bestehen. Es ist auch bevorzugt, dass beide Formteile (1, 2), außer die Verschlusselemente, im Wesentlichen aus einem biokompatiblen Material bestehen. In einer Ausführungsform bestehen beide Formteile (1, 2), außer die Verschlusselemente, im Wesentlichen aus einem biokompatiblen Material, und die Verschlusselemente bestehen nicht aus einem biokompatiblen Material. In einer anderen Ausführungsform bestehen beide Formteile (1, 2) im Wesentlichen aus einem biokompatiblen Material, und die Verschlusselemente bestehen im Wesentlichen aus einem biokompatiblen Material.

Das biokompatible Material ist vorzugsweise ausgewählt aus der Gruppe bestehend aus Kunststoffen, Thermoplasten, Duroplasten, Elastomeren, lichthärtenden Materialien und Mischungen davon. Das biokompatible Material kann beispielsweise ausgewählt sein aus der Gruppe, bestehend aus PVC, Polykarbonat, Polyurethan, Silikon, Polypropylen, Polyethylen, Mischungen davon und Copolymeren davon. Am bevorzugtesten ist das biokompatible Material ein weicher Kunststoff, z. B. Silikon oder Polymethylmethacrylat (PMMA). Die Verwendung eines weichen Kunststoffs hat den Vorteil, dass insbesondere Druckstellen am Ohr vermieden oder verringert werden. In einer besonderen Ausführungsform ist das biokompatible Materialein für den 3-D-Druck geeignetes Material, beispielsweise ein lichthärtendes Material.

Gemäß der Lehre der Erfindung ist es bevorzugt, dass das Verschlusssystem, das die Formteile (1; 2) reversibel miteinander verbindet, so dass die fehlgebildete Ohrmuschel zwischen beide Formteile (1; 2) positioniert werden kann, in die Formteile (1) und (2) integriert ist.

Das Verschlusssystem gemäß Erfindung besteht vorzugsweise aus mindestens zwei Verschlusselementen, wobei das eine Verschlusselement den Gegenpart zum anderen Verschlusselement darstellt. Vorzugsweise enthält die Vorrichtung mehr als ein solches Paar von Verschlusselementen. Die Vorrichtung sollte genügend Paare an Verschlusselementen aufweisen, so dass sichergestellt wird, dass die Formteile (1; 2) an der zu behandelnden Ohrmuschel ausreichend miteinander fixiert werden können. Bevorzugt ist, dass die Vorrichtung mindestens zwei oder mehr, bevorzugter 3 bis 8 solcher Paare enthält, zum Beispiel 3, 4, 5, 6, 7 oder 8. Am bevorzugtesten enthält die Vorrichtung 5 oder 6 Paare an Verschlusselementen.

Gemäß der Erfindung ist es außerdem bevorzugt, dass die jeweiligen VerschlusselementPaare (Paar) so in der Vorrichtung integriert sind (ist), dass das eine Verschlusselement im ersten Formteil (1) integriert ist und das andere Verschlusselement, das den Gegenpart zum ersten Verschlusselement bildet, im zweiten Formteil (2) der erfindungsgemäßen Vorrichtung integriert ist. Insbesondere ist es bevorzugt, dass die Verschlusselemente in den Rändern der Formteile (1; 2), d.h. in den Rändern, die nicht die oben beschriebene Faltenstruktur der Ohrmuschel aufweisen, integriert sind. Dabei sind die Verschlusselemente vorzugsweise so integriert, dass sie sich nicht über die Fläche der im Wesentlichen flächigen Ränder erheben.

Die einzelnen Verschlusselemente können ihrerseits wiederum aus ein oder mehreren Untereinheiten bestehen. Dies hängt von der Art des Verschlusssystems ab.

In einer Ausführungsform der Erfindung ist das Verschlusssystem magnetisch und/oder mechanisch.

Ist das Verschlusssystem magnetisch, so enthält wenigstens ein Verschlusselement ein magnetisches Material oder besteht daraus. Die Materialien der Verschlusselemente werden dabei so gewählt, dass die Verschlusselemente des jeweiligen Verschlusselementpaares sich gegenseitig anziehen.

In einer Ausführungsform enthält das erste Verschlusselement (5) ein magnetisches Material oder besteht daraus, und das zweite Verschlusselement (6) enthält kein magnetisches Material, es enthält aber ein Material, das von dem magnetischen Material des ersten Verschlusselements (5) angezogen wird. In einer anderen Ausführungsform enthält das zweite Verschlusselement (6) ein magnetisches Material oder besteht daraus, und das erste Verschlusselement (5) enthält kein magnetisches Material, es enthält aber ein Material, das von dem magnetischen Material des zweiten Verschlusselements (6) angezogen wird. In einer dritten Ausführungsform enthält das erste Verschlusselement (5) ein magnetisches Material oder besteht daraus, und das zweite Verschlusselement (6) enthält ein magnetisches Material oder besteht daraus, wobei sich das magnetische Material des ersten Verschlusselements (5) und das magnetische Material des zweiten Verschlusselements (6) anziehen.

Das magnetische Material kann zum Bespiel ein ferromagnetisches Metall oder eine entsprechende Metalllegierung sein. Beispiele hierfür sind Kobalt, Nickel, Eisen oder Legierungen daraus, sowie Kobalt-Samarium-Legierungen, Neodym-Eisen-Bor-Legierungen, Aluminium-Nickel-Kobalt-Legierungen, Eisen-Chrom-Kobalt-Legierungen, Kupfer-Nickel-Eisen-Legierungen, Kupfer-Nickel-Kobalt-Legierungen. In bestimmten Fällen ist aufgrund von möglichen Allergien die Verwendung von Nickel nicht bevorzugt. In einer Ausführungsform ist das magnetische Material ein magnetisches Material mit medizinischer Zulassung. In einer Ausführungsform bestehen die Verschlusselemente aus Titanummantelten Magneten. In einer bevorzugten Ausführungsform sind die magnetischen Verschlusselemente von dem biokompatiblem Material (z. B. Silikon) ummantelt.

Ein Beispiel für ein erfindungsgemäß geeignetes mechanisches Verschlusssystem ist ein Klettverschluss. Dabei besteht ein Verschlusselement aus einem synthetischen Faserstreifen mit entsprechenden Schlaufen und das andere Verschlusselement aus einem synthetischen Faserstreifen mit den dazugehörigen flexiblen Widerhäkchen.

Des Weiteren können die Verschlusselementpaare das Prinzip von Druckknöpfen aufweisen, d.h. das eine Verschlusselement enthält eine Vertiefung, das andere Verschlusselement enthält den passenden Kopf dazu. Bei dieser Art von Verschlusssystem können beide Verschlusselemente außerdem magnetische Eigenschaften, wie oben beschrieben, aufweisen.

Die Verschlusselemente sind vorzugsweise so dimensioniert, dass sie die oben beschriebenen zusätzlichen Ränder der Formteile (1; 2) nicht überragen.

Die Form der einzelnen Verschlusselemente ist für die Erfindung nicht wesentlich, jedoch ist es bevorzugt, dass die einzelnen Verschlusselemente eine runde, ovale oder eckige Form aufweisen. Des Weiteren ist bevorzugt, dass die Verschlusselemente, die in einem Formteil integriert sind, über die Gesamtlänge des zusätzlichen Randes des Formteiles gleichmäßig verteilt sind, wobei es besonders bevorzugt ist, wenn sich die einzelnen Verschlusselemente, die in einem Formteil integriert sind, nicht berühren.
In einer anderen Ausführungsform liegen die Verschlusselemente in Form von Bändern oder Streifen in den Formteilen (1; 2) vor, wobei die entsprechenden Bänder bzw. Streifen vorzugsweise über die gesamte Länge der Ränder der Formteile (1; 2) integriert sind. Das Verschlusssystem liegt vorzugsweise außerhalb der Bereiche der Formteile, die die Ohrmuschel abbilden. Vorzugsweise liegt das Verschlusssystem in den Rändern der Formteile. Dadurch werden Druckstellen vermieden. Die erfindungsgemäße Vorrichtung ist in der Regel so ausgestaltet, dass nach Anlegen an das Ohr, dessen Form angepasst werden soll, kein Teil des Ohres zwischen zwei Verschlusselementen eines Verschlusselementpaars liegt.

Durch das erfindungsgemäße Verschlusssystem kann die Vorrichtung einfach an der zu behandelnden Ohrmuschel an- und abgelegt werden. Die Verwendung vom Klebebändern und/oder Klammern ist nicht notwendig.

In manchen Fällen ist es jedoch wünschenswert, dass die Vorrichtung zusätzlich an den Kopf angelegt wird. Hierfür wird vorzugsweise ein Stirnband verwendet. Geeignete Stirnbänder sind im Stand der Technik bekannt. Das Stirnband sollte den Kopf des Säuglings und die an der zu behandelnden Ohrmuschel angelegte erfindungsgemäße Vorrichtung umschließen.

Folglich ist es gemäß einer Ausführungsform der Erfindung bevorzugt, dass die Vorrichtung ein erstes Formteil (1), ein zweites Formteil (2), ein Verschlusssystem, wie oben beschrieben, umfasst bzw. daraus besteht und zusätzlich ein Stirnband enthält.

Die erfindungsgemäße Vorrichtung kann wie folgt hergestellt werden:
a) Gegebenenfalls Erstellen oder Bereitstellen eines Modells der fehlgebildeten Ohrmuschel;
b) Bearbeiten des in Schritt a) erhaltenen Modells, so dass ein Modell einer Ohrmuschel mit gewünschter anatomischer Form erhalten wird;
c) Herstellen eines ersten Formteils, das die Negativform eines Teils der Ohrmuschelvorderseite des in Schritt b) erhaltenen Modells aufweist und wenigstens ein Verschlusselement umfasst; und
d) Herstellen eines zweiten Formteils, das die Negativform eines Teils Ohrmuschelrückseite des in Schritt b) erhaltenen Modells aufweist und wenigstens ein Verschlusselement umfasst;
wobei das erste Formteil und das zweite Formteil reversibel miteinander verbunden werden können, so dass die fehlgebildete Ohrmuschel zwischen beiden Formteilen positioniert werden kann.

Schritt a) und/oder Schritt b) können manuell durchgeführt werden. In einer Ausführungsform wird die zu behandelnde fehlgebildete Ohrmuschel manuell abgeformt, z. B. unter Verwendung eines zur Abformung geeigneten Materials wie Silikon. Aus der so erhaltenen Negativform kann das entsprechende Modell hergestellt werden, beispielsweise durch Ausgießen mit Wachs. Das so hergestellte Modell, z. B. das Wachsmodell, wird anschließend bearbeitet (moduliert), so dass ein Modell mit gewünschter anatomischer Form, d.h. mit der anatomisch korrekten Form, der Ohrmuschel erhalten wird.

Die Schritte a) und/oder b) können auch digital durchgeführt werden, so dass ein Computergeneriertes Modell der fehlgebildeten Ohrmuschel erhalten wird. Insbesondere bevorzugt ist dabei die Verwendung eines 3D-Scans, der die genaue Form der fehlgebildeten Ohrmuschel vermisst (siehe Figur 5). Die Bearbeitung des digitalen Modells in Schritt b) kann ebenfalls am Computer erfolgen, so dass ein digitales Modell der Ohrmuschel mit gewünschter anatomischer Form erhalten wird.

Die Herstellung der Formteile in den Schritten c) und d) kann mittels eines 3D-Druckers erfolgen. In einer anderen Ausführungsform werden die Schritte c) und d) manuell durchgeführt. Beispielsweise kann auf Basis des in Schritt b) erhaltenen Wachsmodells nach Abformung zunächst eine entsprechende Positivform aus Gips hergestellt werden. Auf Basis dieses Gipsmodells kann die erfindungsgemäße Vorrichtung durch Formenbau hergestellt werden.

Bei der Herstellung der Vorrichtung in den Schritten c) und d) des erfindungsgemäßen Verfahrens sollte darauf geachtet werden, dass beide Formteile (1; 2) neben der gewünschten Form und Faltenstruktur der Ohrmuschel den oben beschriebenen zusätzlichen Rand erhalten, damit das erfindungsgemäße Verschlusssystem in die Formteile (1; 2) integriert werden kann.

Durch die oben beschriebene individuelle Herstellung der Vorrichtung, kann die erfindungsgemäße Vorrichtung passgenau an die zu behandelnde Ohrmuschel angepasst werden und somit entstehen keine unerwünschten Druckstellen während der Verwendung der Vorrichtung.

Wie bereits erwähnt, wird die Vorrichtung gemäß Erfindung zur Behandlung von Fehlbildungen bei menschlichen Ohrmuscheln, insbesondere Neugeborenen und Säuglingen verwendet. Besonders bevorzugt ist dabei die Behandlung von Fehlbildungen mit einem Dysplasie Grad von 1.

Dabei wird die individuell hergestellte Vorrichtung mit dem ersten Formteil (1) auf die Vorderseite der zu behandelnden Ohrmuschel gelegt und das zweite Formteil (2) auf die Ohrmuschelrückseite. Beide Formteile (1; 2) werden dann anschließend miteinander durch das erfindungsgemäße Verschlusssystem an der Ohrmuschel fixiert. Durch die Verwendung der erfindungsgemäßen Ohrformungsschiene wird die Fehlbildung der Ohrmuschel behoben.

Die genaue Dauer der Behandlung richtet sich nach Alter des Säuglings zu Beginn der Behandlung und dem Schweregrad der Fehlbildung. Gemäß Erfindung ist eine Behandlungsdauer von 3 Wochen bis zu 6 Monaten, insbesondere von 1 Monat bis zu 4 Monaten, bzw. von 2 bis 3 Monaten bevorzugt.

Die Behandlung sollte sobald als möglich nach der Geburt beginnen, um den größten möglichen Behandlungserfolg zu erzielen. Bevorzugt ist, dass die Behandlung spätestens 6 Monate nach der Geburt, insbesondere 3 Monate nach der Geburt, 1 Monat nach der Geburt, besonders bevorzugt 1 Woche nach der Geburt, beginnt.

### BEISPIELE

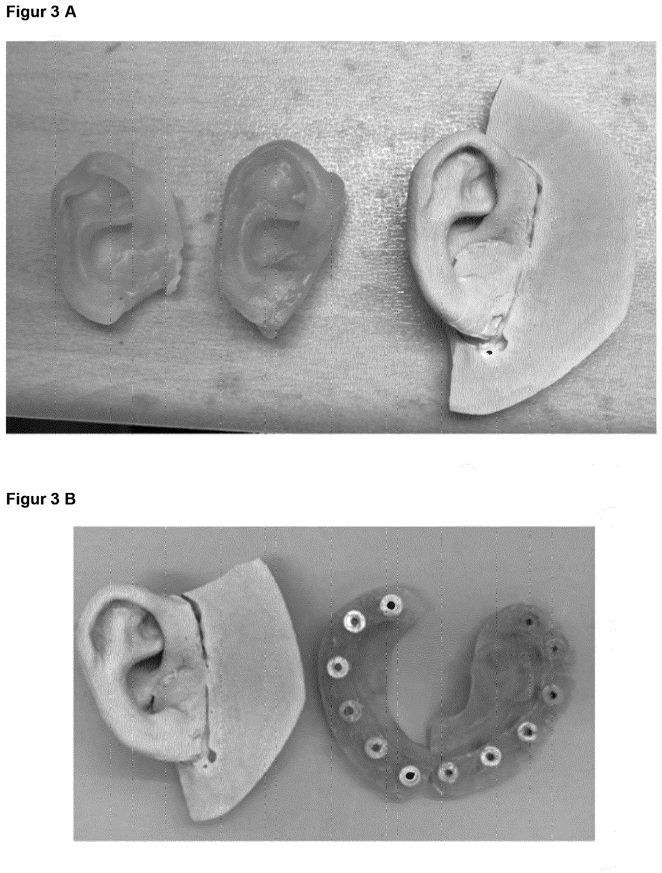

Die erfindungsgemäße Vorrichtung wurde für einen Säugling, der zu Beginn der Behandlung 2 Monate alt war, nach dem erfindungsgemäßen Verfahren hergestellt (siehe Figur 3). Die zu behandelnde Ohrmuschel wies eine Dysplasie von Grad 1 aus.

Nach 3 Wochen Behandlung mittels der erfindungsgemäßen Vorrichtung wies die zu behandelnde Ohrmuschel die anatomisch korrekte Form auf, d.h. die Ohrmuschel hatte nicht länger eine Fehlbildung (siehe Figur 4).

## Patentansprüche

1. Vorrichtung zur Formanpassung einer fehlgebildeten Ohrmuschel eines Menschen, der das erste Lebensjahr noch nicht vollendet hat, umfassend
a. ein erstes Formteil (1) mit einer Vorderseite und einer Rückseite,
b. ein zweites Formteil (2) mit einer Vorderseite und einer Rückseite, und
c. ein Verschlusssystem umfassend mindestens zwei Verschlusselemente (5, 6), wobei das erste Verschlusselement (5) in das erste Formteil integriert ist, und das zweite Verschlusselement (6) in das zweite Formteil integriert ist,
**dadurch gekennzeichnet, dass**
- die Vorderseite des ersten Formteils (1) die Negativform einer gewünschten anatomischen Form eines Teils der Ohrmuschelvorderseite aufweist,
- Die Vorderseite des zweiten Formteils (2) die Negativform einer gewünschten anatomischen Form eines Teils der Ohrmuschelrückseite aufweist, und
- das Verschlusssystem das erste Formteil (1) und das zweite Formteil (2) reversibel miteinander verbinden kann, so dass die fehlgebildete Ohrmuschel zwischen beiden Formteilen (1; 2) positioniert werden kann.

2. Vorrichtung nach Anspruch 1, **dadurch gekennzeichnet, dass** der Teil der Ohrmuschelvorderseite den *Lobulus auriculae,* den *Meatus acusticus* und den *Tragus* nicht umfasst.

3. Vorrichtung nach Anspruch 1 oder 2, **dadurch gekennzeichnet, dass** der Teil der Ohrmuschelvorderseite *Helix*, *Scapha*, und *Anthelix,* und gegebenenfalls weiterhin *Crus superius anthelicis* und *Crus inferius anthelicis* umfasst,

4. Vorrichtung nach einem der Ansprüche 1 bis 3, **dadurch gekennzeichnet, dass** die Formteile (1) und (2) jeweils aus einem Stück bestehen.

5. Vorrichtung nach einem der vorhergehenden Ansprüche, **dadurch gekennzeichnet, dass**
- -das erste Formteil (1) außerhalb des Bereichs, der der Helix entspricht, einen im Wesentlichen flächigen Rand (3) mit mindestens einer Vertiefung (7) oder Erhebung aufweist, und dass
- das zweite Formteil (2) einen im Wesentlichen flächigen Rand (4) mit mindestens einer Vertiefung oder Erhebung (8) aufweist, wobei die Vertiefung im ersten Formteil und die Erhebung im zweiten Formteil komplementär ineinander greifen, wenn die fehlgebildete Ohrmuschel zwischen den beiden Formteilen (1; 2) positioniert wird, oder wobei die Erhebung im ersten Formteil und die Vertiefung im zweiten Formteil komplementär ineinander greifen, wenn die fehlgebildete Ohrmuschel zwischen den beiden Formteilen (1; 2) positioniert wird.

6. Vorrichtung nach einem der vorhergehenden Ansprüche, **dadurch gekennzeichnet, dass** das erste und zweite Formteil (1; 2) im Wesentlichen aus einem biokompatiblen Material bestehen, wobei die Verschlusselemente entweder (i) ebenfalls im Wesentlichen aus einem biokompatiblen Material bestehen oder (ii) nicht aus einem biokompatiblen Material bestehen.

7. Vorrichtung nach einem der vorhergehenden Ansprüche, **dadurch gekennzeichnet, dass** mindestens ein Verschlusselement (5) im Rand (3) des ersten Formteils (1) integriert ist, und mindestens ein Verschlusselement (6) im Rand (4) des zweiten Formteils (2) integriert ist.

8. Vorrichtung nach Anspruch 7, **dadurch gekennzeichnet, dass** das Verschlusselement (5) im Rand (3) des ersten Formteils (1) den Gegenpart zum Verschlusselement (6) im Rand (4) des zweiten Formteils (2) bildet.

9. Vorrichtung nach einem der vorhergehenden Ansprüche, **dadurch gekennzeichnet, dass** das Verschlusssystem magnetisch ist.

10. Vorrichtung nach einem der vorhergehenden Ansprüche, **dadurch gekennzeichnet, dass** alle Verschlusselemente in den Rändern (3; 4) der Formteile (1;2) integriert sind.

11. Vorrichtung nach einem der vorhergehenden Ansprüche, **dadurch gekennzeichnet, dass** die fehlgebildete Ohrmuschel zwischen beiden Formteilen (1; 2) positioniert werden kann, ohne dass sich die Ohrmuschel oder ein anderer Teil des Ohres zwischen zwei Verschlusselementen befindet.

12. Vorrichtung nach einem der vorhergehenden Ansprüche, **dadurch gekennzeichnet, dass** das erste und zweite Formteil (1; 2) mit Ausnahme der Verschlusselemente (5; 6), im Wesentlichen aus einem weichen Kunststoff bestehen.

13. Verfahren zur Herstellung einer Vorrichtung zur Formanpassung einer fehlgebildeten Ohrmuschel nach einem der Ansprüche 1 bis 12, **dadurch gekennzeichnet, dass** das Verfahren folgende Schritte umfasst:
a) Bereitstellen eines Modells der fehlgebildeten Ohrmuschel;
b) Bearbeiten des in Schritt a) erhaltenen Modells, so dass ein Modell einer Ohrmuschel mit gewünschter anatomischer Form erhalten wird;
c) Herstellen eines ersten Formteils, das die Negativform eines Teils der Ohrmuschelvorderseite des in Schritt b) erhaltenen Modells aufweist und wenigstens ein Verschlusselement umfasst; und
d) Herstellen eines zweiten Formteils, das die Negativform eines Teils Ohrmuschelrückseite des in Schritt b) erhaltenen Modells aufweist und wenigstens ein Verschlusselement umfasst;
wobei das erste Formteil und das zweite Formteil reversibel miteinander verbunden werden können, so dass die fehlgebildete Ohrmuschel zwischen beiden Formteilen positioniert werden kann.

14. Verfahren nach Anspruch 13, **dadurch gekennzeichnet, dass** Schritt a) durch einen 3D-Scan durchgeführt wird.

15. Verfahren nach Anspruch 13 oder 14, **dadurch gekennzeichnet, dass** die Schritte c) und/oder d) die Verwendung eines 3D-Druckers umfassen.
